# EUROPEAN PATENT APPLICATION

(11) **EP 4 606 300 A1**
(43) Date of publication of application: **27.08.2025**
(21) Application number: 25152887.3
(22) Date of filing: 20.01.2025
(51) Int. Cl.: A61B 5/021, A61B 5/117, A61B 5/00, A61B 5/024

(54) **APPARATUS, METHOD, AND COMPUTER PROGRAM**

(30) Priority: 22.02.2024 GB 202402508
(71) Applicant: Nokia Technologies Oy, 02610 Espoo (FI)
(72) Inventor: FERLINI, Andrea, Cambridge (GB); ROMERO, Julia, Boulder (US); MONTANARI, Alessandro, Cambridge (GB)
(74) Representative: Nokia EPO representatives

(57) **Abstract**

The disclosure relates to a method comprising: receiving a request to authenticate a user; determining a pulse time based on a timestamp associated with a feature within a first photoplethysmography waveform sensed by a first wearable device on a first region of the user and a timestamp associated with the feature within a second photoplethysmography waveform sensed by a second wearable device on a second region of the user; and authenticating the user based on the pulse time

## Description

### Field of the disclosure

The present disclosure relates to an apparatus, a method, and a computer program for authenticating a user using biometric data.

### Background

Authenticating a user using biometric data may be used to grant access to a user equipment. Typically, the user equipment may receive biometric data of the user and determine whether the biometric data of the user matches reference biometric data stored in a databased. If the biometric data of the user matches the reference biometric data stored in the databased, the user may be authenticated and the user equipment may grant access to the user equipment. If the biometric data of the user matches the reference biometric data stored in the databased, the user may not be authenticated and the user equipment deny access to the user equipment.

### Summary

According to an aspect there is provided a method comprising: determining a pulse time based on a timestamp associated with a feature within a first waveform sensed by a first wearable device on a first region of the user and a timestamp associated with the feature within a second waveform sensed by a second wearable device on a second region of the user; and authenticating the user based on the pulse time.

The first waveform may be a first photoplethysmography waveform and the second waveform may be a second photoplethysmography waveform.

The first region and the second region may be located along a same artery branch of the user. The pulse time may be a pulse transit time.

The first region and the second region may be located along different artery branches of the user. The pulse time may be a pulse arrival time.

The feature may comprise at least one of: a start of beat; a systolic peak; a dicrotic notch; a diastolic peak; or an end of beat.

The method may comprise: receiving the first waveform; determining the timestamp associated with the feature within the first waveform; receiving the second waveform; and determining the timestamp associated with the feature within the second photoplethysmography waveform;

The method may comprise: receiving the timestamp associated with the feature within the first waveform; and receiving the timestamp associated with the feature within the second waveform.

Authenticating the user based on the pulse time may comprise: determining that the pulse time matches a reference pulse time; and authenticating the user based on the pulse time matching the reference pulse time.

The method may comprise: determining the reference pulse time based on a timestamp of the feature within a previous first waveform sensed by the first wearable device on the first region of the user and a timestamp of the feature within a previous second waveform sensed by the second wearable device on the second region of the user.

The method may comprise: updating the reference pulse time based on the pulse time.

The first wearable device and the second wearable device may be time synchronized.

The method may comprise: determining a first inter-beat interval based on a timestamp associated with the feature within the first waveform wearable device and a timestamp associated with a consecutive feature within the first waveform wearable device; determining a second inter-beat interval based on a timestamp associated with a feature within the second waveform sensed by the second wearable device on the second region of the user and a timestamp associated with a consecutive feature within the second waveform wearable device; and authenticating the user based on the first inter-beat interval and the second inter-beat interval.

Authenticating the user based on the first inter-beat interval and the second inter-beat interval may comprise: determining that the first inter-beat interval matches the second inter-beat interval; and authenticating the user based the first inter-beat interval matching the second inter-beat interval.

The method may comprise: unlocking the second wearable device.

At least one of the first wearable device and the second wearable device may comprise: a phone; a watch; a ring; a wrist band; an arm band; a chest strap; an earbud; a garment; a headset; a head mounted visor; or glasses.

The method may comprise receiving a request to authenticate a user;

The method may be performed by a user equipment.

The user equipment may be a smartphone.

According to an aspect there is provided an apparatus comprising: means for determining a pulse time based on a timestamp associated with a feature within a first waveform sensed by a first wearable device on a first region of the user and a timestamp associated with the feature within a second waveform sensed by a second wearable device on a second region of the user; and means for authenticating the user based on the pulse time.

The first waveform may be a first photoplethysmography waveform and the second waveform may be a second photoplethysmography waveform.

The first region and the second region may be located along a same artery branch of the user. The pulse time may be a pulse transit time.

The first region and the second region may be located along different artery branches of the user. The pulse time may be a pulse arrival time.

The feature may comprise at least one of: a start of beat; a systolic peak; a dicrotic notch; a diastolic peak; or an end of beat.

The apparatus may comprise: means for receiving the first waveform; determining the timestamp associated with the feature within the first waveform; means for receiving the second waveform; and means for determining the timestamp associated with the feature within the second photoplethysmography waveform;

The apparatus may comprise: means for receiving the timestamp associated with the feature within the first waveform; and means for receiving the timestamp associated with the feature within the second waveform.

Authenticating the user based on the pulse time may comprise: determining that the pulse time matches a reference pulse time; and authenticating the user based on the pulse time matching the reference pulse time.

The apparatus may comprise: means for determining the reference pulse time based on a timestamp of the feature within a previous first waveform sensed by the first wearable device on the first region of the user and a timestamp of the feature within a previous second waveform sensed by the second wearable device on the second region of the user.

The apparatus may comprise: means for updating the reference pulse time based on the pulse time.

The first wearable device and the second wearable device may be time synchronized.

The apparatus may comprise: means for determining a first inter-beat interval based on a timestamp associated with the feature within the first waveform wearable device and a timestamp associated with a consecutive feature within the first waveform wearable device; means for determining a second inter-beat interval based on a timestamp associated with a feature within the second waveform sensed by the second wearable device on the second region of the user and a timestamp associated with a consecutive feature within the second waveform wearable device; and means for authenticating the user based on the first inter-beat interval and the second inter-beat interval.

Authenticating the user based on the first inter-beat interval and the second inter-beat interval may comprise: determining that the first inter-beat interval matches the second inter-beat interval; and authenticating the user based the first inter-beat interval matching the second inter-beat interval.

The apparatus may comprise: unlocking the second wearable device.

At least one of the first wearable device and the second wearable device may comprise: a phone; a watch; a ring; a wrist band; an arm band; a chest strap; an earbud; a garment; a headset; a head mounted visor; or glasses.

The apparatus may comprise receiving a request to authenticate a user;

The apparatus may be a user equipment.

The user equipment may be a smartphone.

According to an aspect there is provided an apparatus comprising at least one processor and at least one memory storing instructions that, when executed by the at least one processor, cause the apparatus at least to perform: determining a pulse time based on a timestamp associated with a feature within a first waveform sensed by a first wearable device on a first region of the user and a timestamp associated with the feature within a second waveform sensed by a second wearable device on a second region of the user; and authenticating the user based on the pulse time.

According to an aspect there is provided an apparatus comprising circuitry configured to perform: determining a pulse time based on a timestamp associated with a feature within a first waveform sensed by a first wearable device on a first region of the user and a timestamp associated with the feature within a second waveform sensed by a second wearable device on a second region of the user; and authenticating the user based on the pulse time.

According to an aspect there is provided a computer program comprising computer executable code which when run on at least one processor is configured to perform: determining a pulse time based on a timestamp associated with a feature within a first waveform sensed by a first wearable device on a first region of the user and a timestamp associated with the feature within a second waveform sensed by a second wearable device on a second region of the user; and authenticating the user based on the pulse time.

According to an aspect, there is provided a computer readable medium comprising program instructions stored thereon for performing at least one of the above methods.

According to an aspect, there is provided a non-transitory computer readable medium comprising program instructions stored thereon for performing at least one of the above methods.

According to an aspect, there is provided a non-volatile tangible memory medium comprising program instructions stored thereon for performing at least one of the above methods.

In the above, many different aspects have been described. It should be appreciated that further aspects may be provided by the combination of any two or more of the aspects described above.

Various other aspects are also described in the following detailed description and in the attached claims.

### List of abbreviations

- CPU:: Computing Processing Unit
- IBI:: Inter-Beat Interval
- OTP:: One Time Password
- PAT:: Pulse Arrival Time
- PPG:: Photoplethysmography
- PT:: Pulse Time
- PTT:: Pulse Time Interval
- RAM:: Random Access Memory
- ROM:: Read Only Memory
- UE:: User equipment

### Brief Description of the Figures

Embodiments will now be described, by way of example only, with reference to the accompanying Figures in which:
Fig. 1 shows a schematic representation of a user equipment;
Fig. 2 shows a schematic representation of a photoplethysmography waveform;
Fig. 3 shows a schematic representation of a first photoplethysmography waveform sensed by a first wearable device and a second photoplethysmography waveform sensed by a second wearable device;
Fig. 4 shows a signaling diagram of a process for authenticating a user using biometric data, where the process is performed by a user equipment, a first wearable device and a second wearable device ;
Fig. 5 shows a block diagram of method for authenticating a user using biometric data; and
Fig. 6 shows a schematic representation of a non-volatile memory medium storing instructions which when executed by a processor allow a processor to perform one or more of the steps of the methods of Fig. 5.

### Detailed Description of the Figures

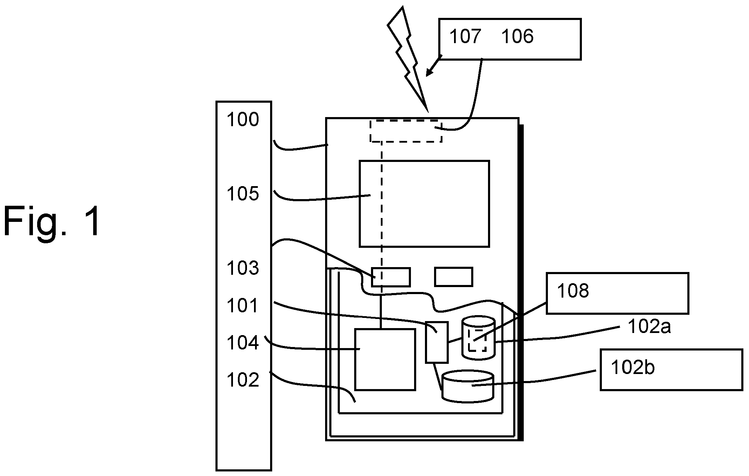

Figure 1 illustrates an example of a user equipment (UE) 100. The UE 100 may be provided by any device capable of sending and receiving radio signals. A non-limiting example may comprise a smart phone or a tablet. The UE 100 may provide communication of data for carrying communications. The communications may be one or more of voice, electronic mail (email), text message, multimedia, data, machine data and so on.

The UE 100 may receive signals over an air or radio interface 107 via appropriate apparatus for receiving and may transmit signals via appropriate apparatus for transmitting radio signals. In Figure 1 transceiver apparatus is designated schematically by block 106. The transceiver apparatus 106 may be provided for example by means of a radio part and associated antenna arrangement. The antenna arrangement may be arranged internally or externally to the mobile device.

The UE 300 may be provided with at least one processor 101, at least one memory ROM 102a, at least one RAM 102b and other possible components 103 for use in software and hardware aided execution of tasks it is designed to perform, including control of access to and communications with access systems and other communication devices. The at least one processor 101 is coupled to the RAM 102b and the ROM 102a. The at least one processor 101 may be configured to execute an appropriate software code 108. The software code 108 may for example allow to perform one or more of the present aspects. The software code 108 may be stored in the ROM 102a.

The processor, storage and other relevant control apparatus can be provided on an appropriate circuit board and/or in chipsets. This feature is denoted by reference 104. The device may optionally have a user interface such as keypad 105, touch sensitive screen or pad, combinations thereof or the like. Optionally one or more of a display, a speaker and a microphone may be provided depending on the type of the device.

One or more aspects of this disclosure relates to techniques for authenticating a user using biometric data.

A UE may be paired to a first wearable device and a second wearable device (e.g., a watch, a ring, a wrist band, an arm band, a chest strap, an earbud, a garment, a headset, a head mounted visor or glasses). The first wearable device and the second wearable may include sensors capable of sensing cardiac waveforms. In the following, the first wearable device and the second wearable may include photoplethysmography (PPG) sensors but it will be understood that alternative sensors may be used as long as they are capable of sensing a cardiac waveforms (e.g., a microphones).

It will be understood that the first wearable device and the second wearable may include same sensors (i.e., the first wearable device and the second wearable may both include PPG sensors or may both include microphones). Alternatively, the first wearable device and the second wearable may include different sensors (i.e., the first wearable device may include a PPG sensor and the second wearable may include a microphone, or vice versa).

A PPG sensor may measure blood volume changes in tissues. When light is shone, an absorption pattern changes whenever blood is being pumped. A PPG waveform sensed by a PPG sensor may comprise a pulse whenever "freshly pumped" blood flows in the region of a user where the PPG sensor is placed.

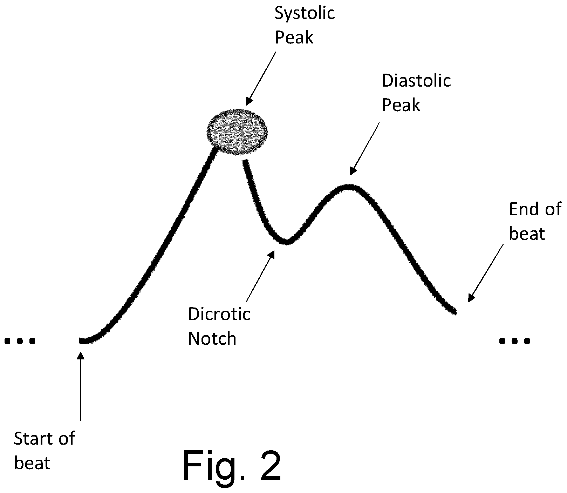

Fig. 2 shows a schematic representation of photoplethysmography waveform. The photoplethysmography waveform comprises multiple pulses (here only one peak is represented). Each pulse may comprise features (e.g., start of beat, systolic peak, dicrotic notch, diastolic peak and end of beat). Each feature may be associated with a timestamp.

If PPG sensors are placed in different regions of the user, the PPG waveforms sensed by the multiple PPG sensors would comprise peaks at slightly different times depending on the distance between the heart of the user and the different regions of the user. This may be due to the time it takes the "freshly pumped" blood to travel from the heart of the user to the different regions of the user. For instance, it would take less time for the "freshly pumped" blood to travel from the heart of the user to the wrist of the user than from the heart of the user to the fingers of the user.

The time for the "freshly pumped" blood to travel from the heart of the user to the different regions of the user may depend on the blood viscosity, the blood velocity, and the blood has to travel. The blood velocity may be affected by the blood pressure and the arterial elasticity, which are highly individual to each person's physiology and age. The shape of a pulse shape may depend on the arterial stiffness, the arterial resistance, and the ventricular ejection.

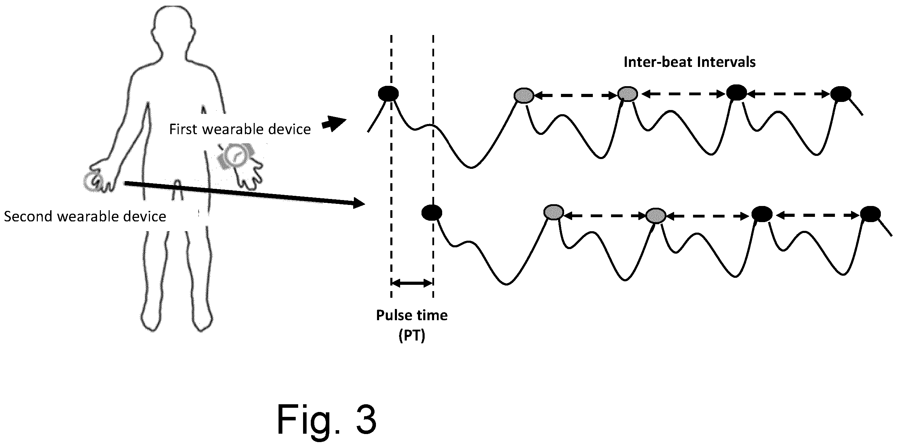

Fig. 3 shows a schematic representation of a first PPG waveform sensed by a first wearable device (e.g., smart watch) and a second PPG waveform sensed by a second wearable device (e.g., smart ring).

The first PPG waveform and the second PPG waveform may be shifted out of phase.

The time difference between the time stamp associated with a feature (e.g., systolic peak) within the first PPG waveform and the time stamp associated with the feature (e.g., systolic peak) within the second PPG waveform for a same heartbeat (i.e., same cardiac cycle) may be referred to as a pulse time (PT).

When the first wearable device and the second wearable device are placed along a same arterial branch of the user, the time difference between the time stamp associated with a feature (e.g., systolic peak) within the first PPG y waveform and the time stamp associated with the feature (e.g., systolic peak) within the second PPG waveform may be referred to as a pulse transit time (PTT).

When the first wearable device and the second wearable device are placed along different arterial branches of the user, the time difference between the time stamp associated with a feature (e.g., systolic peak) within the first PPG waveform and the time stamp associated with the feature (e.g., systolic peak) within the second PPG waveform may be referred to as a pulse arrival time (PAT).

The PT may depend on the body, tissue and blood-vessel composition of the user. The PT may depend on the user's vascular elasticity, heart rate, blood pressure and vasoconstriction. As a result, the PT may be unique to the user.

It will be understood that the PT may be determined by the UE using a minimum amount of resources (e.g., battery or CPU).

In an example, the first wearable device may locally process the first PPG waveform to determine a feature (e.g., systolic peak) within the first PPG waveform and a timestamp associated with the feature (e.g., systolic peak) within the first PPG waveform.

The first wearable device may transmit, to the UE, the timestamp associated with the feature (e.g., systolic peak) within the first PPG waveform.

The second wearable device may locally process the second PPG waveform to determine a feature (e.g., systolic peak) within the second PPG waveform and a timestamp associated with the feature (e.g., systolic peak) within the second PPG waveform.

The second wearable device may transmit, to the UE, the timestamp associated with the feature (e.g., systolic peak) within the second PPG waveform.

The UE may determine the PT based on the timestamp associated with the feature (e.g., systolic peak) within the first PPG waveform and the timestamp associated with the feature (e.g., systolic peak) within the second PPG waveform. The UE may subtract the timestamp associated with the feature (e.g., systolic peak) within the first PPG waveform and the timestamp associated with the feature (e.g., systolic peak) within the second PPG waveform (e.g., PT= T_{systolic peak_first device}- T_{systolic peak_second device}).

In another example, the first wearable device may transmit, to the UE, the first PPG waveform. The UE may process the first PPG waveform to determine a feature (e.g., systolic peak) within the first PPG waveform and a timestamp associated with the feature (e.g., systolic peak) within the first PPG waveform.

The second wearable device may transmit, to the UE the second PPG waveform.

The UE may determine a feature (e.g., systolic peak) within the second PPG waveform and a timestamp associated with the feature (e.g., systolic peak) within the second PPG waveform.

The UE may determine the PT based on the timestamp associated with the feature (e.g., systolic peak) within the first PPG waveform and the timestamp associated with the feature (e.g., systolic peak) within the second PPG waveform. The UE may subtract the timestamp associated with the feature (e.g., systolic peak) within the first PPG waveform and the timestamp associated with the feature (e.g., systolic peak) within the second PPG waveform (e.g., PT= T_{systolic peak_first device}- T_{systolic peak_second device}).

One or more aspects of this disclosure relates to techniques for authenticating a user using the PT . The PT may be difficult (if not impossible) to be forged, and any attempt by a malicious user to influence his or her cardiovascular system to forge the PT is likely to fail.

The time difference between the time stamp associated with a feature (e.g., systolic peak) within the first PPG waveform and the time stamp associated with a consecutive feature (e.g., systolic peak) within the first PPG waveform for consecutive heartbeats (i.e., consecutive cardiac cycles) may be referred to as a first inter-beat interval (IBI).

The time difference between the time stamp associated with a feature (e.g., systolic peak) within the second PPG waveform and the time stamp associated with a consecutive feature (e.g., systolic peak) within the second PPG waveform for consecutive heartbeats (i.e., consecutive cardiac cycles) may be referred to as a second inter-beat interval (IBI).

The first IBI and the second IBI may be identical.

It will be understood that the first IBI may be determined by the first wearable device or the UE using a minimum amount of resources (e.g., battery or CPU).

In an example, the first wearable device may locally process the first PPG waveform to determine consecutive features (e.g., systolic peaks) within the first PPG waveform and timestamps associated with the consecutive features (e.g., systolic peaks) within the first PPG waveform. The first wearable device may transmit, to the UE, the timestamps associated with the consecutive features (e.g., systolic peaks) within the first PPG waveform. The UE may determine the first IBI based on the timestamps associated with the consecutive features (e.g., systolic peaks) within the first PPG waveform. The UE may subtract the timestamps associated with the consecutive features (e.g., systolic peaks) within the first PPG waveform (e.g., first IBI = _{Tsystolic peak} i,_ first wearable device - T systolic _{peak_i+1_first} wearable device).

In another example, the first wearable device may transmit, to the UE, the first PPG waveform. The UE may process the first PPG waveform to determine consecutive features (e.g., systolic peaks) within the first PPG waveform and timestamps associated with the consecutive features (e.g., systolic peaks) within the first PPG waveform. The UE may determine the first IBI based on the timestamps associated with the consecutive features (e.g., systolic peaks) within the first PPG waveform. The UE may subtract the timestamps associated with the consecutive features (e.g., systolic peaks) within the first PPG waveform (e.g., first IBI = _{T systolic peak i,_ first wearable} device - T systolic _{peak_i+1_first} wearable device).

It will be understood that the second IBI may be determined by the second wearable device or the UE using a minimum amount of resources (e.g., battery or CPU).

In an example, the second wearable device may locally process the second PPG waveform to determine consecutive features (e.g., systolic peaks) within the second PPG waveform and timestamps associated with the consecutive features (e.g., systolic peaks) within the second PPG waveform. The second wearable device may transmit, to the UE, the timestamps associated with the consecutive features (e.g., systolic peaks) within the second PPG waveform. The UE may determine the second IBI based on the timestamps associated with the consecutive features (e.g., systolic peaks) within the second PPG waveform. The UE may subtract the timestamps associated with the consecutive features (e.g., systolic peaks) within the second PPG waveform (e.g., second IBI = _{T systolic peak i,_second wearable device} - T _{systolic peak_i+1_second wearable} device).

In another example, the second wearable device may transmit, to the UE, the second PPG waveform. The UE may process the second PPG waveform to determine consecutive features (e.g., systolic peaks) within the second PPG waveform and timestamps associated with the consecutive features (e.g., systolic peaks) within the second PPG waveform. The UE may determine the second IBI based on the timestamps associated with the consecutive features (e.g., systolic peaks) within the second PPG waveform. The UE may subtract the timestamps associated with the consecutive features (e.g., systolic peaks) within the second PPG waveform (e.g., second IBI = Tsystolic peak i,_ second wearable device - T systolic peak_i+1 second wearable device).

One or more aspects of this disclosure relates to techniques for authenticating a user using the first IBI and the second IBI. The first IBI and the second IBI may be difficult (if not impossible) to be forged, and any attempt by a malicious user to influence his or her cardiovascular system to forge the first IBI and the second IBI is likely to fail.

It will be understood that existing wearable devices including PPG sensors are already configured to perform detection algorithms to detect features (e.g., systolic peaks) in PPG waveforms in order to measure a heart rate. As a result, the determination of the first IBI and the second IBI by such existing wearable devices may be implanted with reduced complexity by carrying out a simple subtraction.

It will be understood that the first IBI and the second IBI may keep changing over time. The first IBI and the second IBI may be used in addition to the PT (e.g., as a one-time password additional authentication factor) or instead of the PT (e.g., should time synchronisation of the first wearable device and the second wearable device with the UE not succeed).

Our invention leverages IBI (which is strictly unique to each and every individual and keeps on changing over time) as an additional authentication layer (think of it as a one-time-password second authentication factor - OTP 2FA) or should the time sync not succeed for any reason.

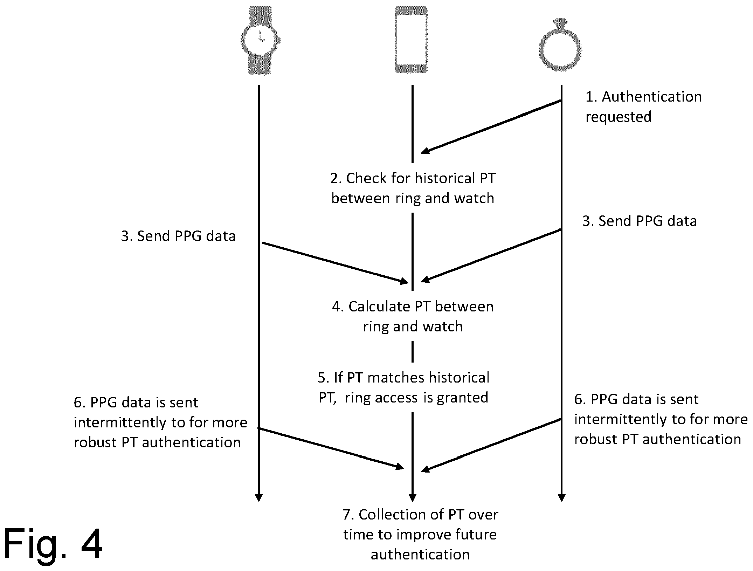

Fig. 4 shows a signaling diagram of a process for authenticating a user using biometric data.

In a calibration phase (i.e., enrolment phase), a user may wear a first wearable device (e.g., smart watch) and a second wearable device (e.g., smart ring). The first wearable device and the second wearable device may be paired with a UE (e.g., smart phone). The first wearable device and the second wearable device may communicate with the UE via Bluetooth low energy. The UE may act as sink and aggregator for data transmitted by the first wearable device and the second wearable device.

The first wearable device and the second wearable device may be time synchronized with the UE.

Synchronization may be necessary because the determination of a PT is based on timestamps recorded by the first wearable device and the second wearable device. The first wearable device and the second wearable device may experience clock drift over time and may be synchronized to the UE at the start of every authentication attempt. A precise time synchronization technique (low standard deviation) may be used because the PT is directly influenced by the precision of the time synchronization.

Concretely, if time synchronization variation over time TS1 - TS2 = 50 µs, then PT variation over time PT1 - PT2 = 50 µs + v + *ε*, where v is error due to physiology change over time and *ε* is an error term. A large value of PT variation over time PT1 - PT2 may lead to an incorrect failed authentication, so it may be important to minimize the time synchronization variation over time TS1 - TS2 (maximize the precision). The accuracy of the time synchronization may be less important than the consistency of the time synchronization.

The accuracy and consistency of the time synchronization may depend on hardware and time synchronization technique used. For reference, existing time synchronization techniques based on Bluetooth energy may achieve an error of less than 1 µs with a standard deviation of less than 50 ns, which is better than what would be needed to avoid incorrect failed authentication.

The first wearable device may comprise a user input interface. The user may provide a valid personal identification number or fingerprint to the first wearable device via the user input interface. The first wearable device may be unlocked (e.g., the user and the UE are granted full access to the first wearable device).

By contrast, the user may comprise a user input interface. The user may not provide a valid personal identification number or fingerprint to the first wearable device via a user input interface. The second wearable device may be locked (i.e., the user and the UE are granted partial access to the second wearable device).

The second wearable device may transmit a request, to the UE, to calibrate the second wearable device.

The UE may accept the request to calibrate the second wearable device.

The first wearable device may transmit, to the UE, the first PPG waveform or a timestamp associated with a feature (e.g., systolic peak) within the first PPG waveform. The second wearable device may transmit the second PPG waveform or a timestamp associated with the feature (e.g., systolic peak) within the second PPG waveform.

The UE may determine a reference PT based on the first PPG waveform and the second PPG waveform or based on the timestamp associated with the feature (e.g., systolic peak) within the first PPG waveform and the timestamp associated with the feature (e.g., systolic peak) within the second PPG waveform (as explained above).

The UE may compare a first IBI based on the first PPG waveform and a second IBI based on the first PPG waveform and the second PPG waveform (as explained above). The UE may determine that the first IBI matches the second IBI.

The UE may store the reference PT associated with information identifying the first wearable device and the second wearable device (e.g., <PT, identifier of pair formed by first wearable device and second wearable device >). The reference PT may be used by the UE to authenticate the user in a simple and speedy manner in a use phase as will be apparent further below.

The first wearable device and the second wearable device may be unpaired with the UE.

In an implementation, the calibration phase may not be limited to the first wearable device placed on a first region of the user and the second wearable device place on a second region of the user and storing a single reference PT. The calibration phase may be also be performed for the first wearable device placed on a first region of the user and additional wearable devices (e.g., ear bud, chest strap) placed on additional regions of the user and storing additional reference PTs.

It will be understood that the more wearable devices the user wears the more accurate the authentication of the user may be and therefore the authentication of the user may be used for more safety critical applications.

In a use phase (i.e., authentication phase), the user may wear the first wearable device (e.g., smart watch) and the second wearable device (e.g., smart ring). The first wearable device and the second wearable device may be paired with the UE (e.g., smart phone). The first wearable device and the second wearable device may communicate with the UE via Bluetooth low energy. The UE may act as sink and aggregator for data transmitted by the first wearable device and the second wearable device.

The first wearable device and the second wearable device may be time synchronized with the UE.

The user may provide a valid personal identification number or fingerprint to the first wearable device via the user input interface. The first wearable device may be unlocked (e.g., the user and the UE are granted full access to the first wearable device).

The user may not provide a valid personal identification number or fingerprint to the first wearable device via a user input interface. The second wearable device may be locked (i.e., the user and the UE are granted partial access to the second wearable device).

At step 1, the second wearable device may transmit a request, to the UE, to authenticate the user.

The UE may accept the request to authenticate the user.

At step 2, the UE may determine that the UE stored a reference PT associated information identifying the first wearable device and the second wearable device (e.g., <PT, identifier of pair formed by first wearable device and second wearable device>) during the calibration phase.

At step 3, the first wearable device may transmit, to the UE, the first PPG waveform or a timestamp associated with a feature (e.g., systolic peak) within the first PPG waveform. The second wearable device may transmit the second PPG waveform or a timestamp associated with the feature (e.g., systolic peak) within the second PPG waveform.

At step 4, the UE may determine a PT based on the first PPG waveform and the second PPG waveform or based on the timestamp associated with the feature (e.g., systolic peak) within the first PPG waveform and the timestamp associated with the feature (e.g., systolic peak) within the second PPG waveform (as explained above).

At step 5, the UE may compare the PT with the reference PT stored by the UE associated with information identifying the first wearable device and the second wearable device (e.g., <PT, identifier of pair formed by first wearable device and second wearable device>) during the calibration phase. The UE may determine that the PT matches the reference PT.

The UE may compare a first IBI based on the first PPG waveform and a second IBI over a number of heartbeats based on the first PPG waveform and the second PPG waveform (as explained above). The number of heartbeats may be equal to or greater than two.

If the UE determine that the first IBI matches the second IBI over the number of heartbeats, the UE may authenticate the user. The user has worn the second wearable device before and is legitimate. The UE may unlock the second wearable device (e.g., the user and the UE are granted full access to the first wearable device).

If the UE determine that the first IBI does not match the second IBI over the number of heartbeats, the UE may not authenticate the user. The user has not worn the second wearable device before and is not legitimate. The UE may not unlock the second wearable device (e.g., the user and the UE are not granted full access to the first wearable device). The UE may raise a warning and may ask the user to authenticate himself/herself via another authentication technique.

Additionally or alternatively, the UE may compare a first biomarker (e.g., first heartbeat or first heartbeat variation) sensed by the first wearable device and a second biomarker (e.g., second heartbeat or second heartbeat variation) sensed by the second wearable device.

If the first biomarker (e.g., first heartbeat or first heartbeat variation) sensed by the first wearable device matches the second biomarker (e.g., second heartbeat or second heartbeat variation) sensed by the second wearable device, the UE may authenticate the user. The user has worn the second wearable device before and is legitimate. The UE may unlock the second wearable device (e.g., the user and the UE are granted full access to the first wearable device).

If the first biomarker (e.g., first heartbeat or first heartbeat variation) sensed by the first wearable device does not match the second biomarker (e.g., second heartbeat or second heartbeat variation) sensed by the second wearable device, the UE may not authenticate the user. The user has not worn the second wearable device before and is not legitimate. The UE may not unlock the second wearable device (e.g., the user and the UE are not granted full access to the first wearable device). The UE may raise a warning and may ask the user to authenticate himself/herself via another authentication technique.

The UE may use the PT to update the reference PT stored by the UE associated with information identifying the first wearable device and the second wearable device (e.g., <PT, identifier of pair formed by first wearable device and second wearable device>). This may ensure optimal performance and robustness over time.

At step 6, the first wearable device may intermittently (e.g., periodically) transmit, to the UE, new first PPG waveform or a timestamp associated with a feature (e.g., systolic peak) within the new first PPG waveform. The second wearable device may intermittently (e.g., periodically) transmit a new second PPG waveform or a timestamp associated with the feature (e.g., systolic peak) within the new second PPG waveform.

At step 7, the UE may determine a new PT based on the new first PPG waveform and the new second PPG waveform or based on the timestamp associated with the feature (e.g., systolic peak) within the new first PPG waveform and the timestamp associated with the feature (e.g., systolic peak) within the new second PPG waveform (as explained above).

The UE may use the new PT to update the reference PT stored by the UE associated with information identifying the first wearable device and the second wearable device (e.g., <PT, identifier of pair formed by first wearable device and second wearable device>). This may ensure optimal performance and robustness over time.

It will be understood that, although aspects relating to authenticating the user using a PT have been disclosed in combination with aspects relating to authenticating the user using IBIs, these aspects may be implemented independently.

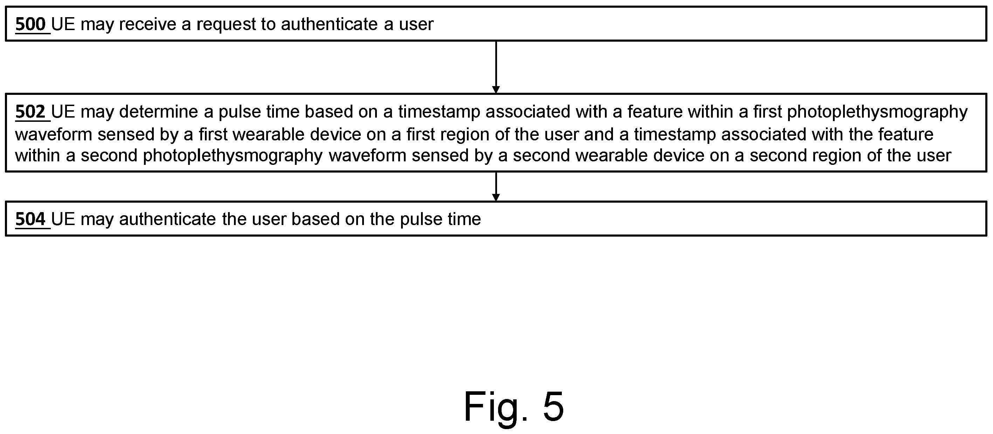

Fig. 5 shows a block diagram of method for authenticating a user using biometric data.

At step 500, a UE may receive a request to authenticate a user.

At step 502, the UE may determine a pulse time based on a timestamp associated with a feature within a first photoplethysmography waveform sensed by a first wearable device on a first region of the user and a timestamp associated with the feature within a second photoplethysmography waveform sensed by a second wearable device on a second region of the user.

At step 504, the UE may authenticate the user based on the pulse time.

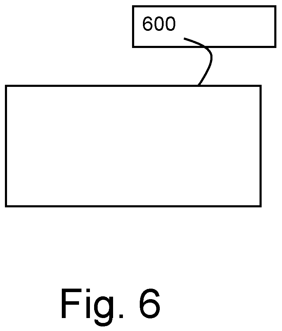

Fig. 6 shows a schematic representation of non-volatile memory media 600 storing instructions which when executed by a processor allow the processor to perform one or more of the steps of the methods of Fig. 5.

It is noted that while the above describes example embodiments, there are several variations and modifications which may be made to the disclosed solution without departing from the scope of the present invention.

The embodiments may thus vary within the scope of the attached claims. In general, some embodiments may be implemented in hardware or special purpose circuits, software, logic or any combination thereof. For example, some aspects may be implemented in hardware, while other aspects may be implemented in firmware or software which may be executed by a controller, microprocessor or other computing device, although embodiments are not limited thereto. While various embodiments may be illustrated and described as block diagrams, flow charts, or using some other pictorial representation, it is well understood that these blocks, apparatus, systems, techniques or methods described herein may be implemented in, as non-limiting examples, hardware, software, firmware, special purpose circuits or logic, general purpose hardware or controller or other computing devices, or some combination thereof.

The embodiments may be implemented by computer software stored in a memory and executable by at least one data processor of the involved entities or by hardware, or by a combination of software and hardware. Further in this regard it should be noted that any procedures, e.g., as in Fig. 5, may represent program steps, or interconnected logic circuits, blocks and functions, or a combination of program steps and logic circuits, blocks and functions. The software may be stored on such physical media as memory chips, or memory blocks implemented within the processor, magnetic media such as hard disk or floppy disks, and optical media such as for example DVD and the data variants thereof, CD.

The memory may be of any type suitable to the local technical environment and may be implemented using any suitable data storage technology, such as semiconductor-based memory devices, magnetic memory devices and systems, optical memory devices and systems, fixed memory and removable memory. The data processors may be of any type suitable to the local technical environment, and may include one or more of general purpose computers, special purpose computers, microprocessors, digital signal processors (DSPs), application specific integrated circuits (ASIC), gate level circuits and processors based on multi-core processor architecture, as non-limiting examples.

Alternatively or additionally some embodiments may be implemented using circuitry. The circuitry may be configured to perform one or more of the functions and/or method steps previously described. That circuitry may be provided in the base station and/or in the communications device.

As used in this application, the term "circuitry" may refer to one or more or all of the following:
(a) hardware-only circuit implementations (such as implementations in only analogue and/or digital circuitry);
(b) combinations of hardware circuits and software, such as:
   (i) a combination of analogue and/or digital hardware circuit(s) with software/firmware and
   (ii) any portions of hardware processor(s) with software (including digital signal processor(s)), software, and memory(ies) that work together to cause an apparatus, such as the communications device or base station to perform the various functions previously described; and
(c) hardware circuit(s) and or processor(s), such as a microprocessor(s) or a portion of a microprocessor(s), that requires software (e.g., firmware) for operation, but the software may not be present when it is not needed for operation.

This definition of circuitry applies to all uses of this term in this application, including in any claims. As a further example, as used in this application, the term circuitry also covers an implementation of merely a hardware circuit or processor (or multiple processors) or portion of a hardware circuit or processor and its (or their) accompanying software and/or firmware. The term circuitry also covers, for example integrated device.

The foregoing description has provided by way of exemplary and non-limiting examples a full and informative description of some embodiments However, various modifications and adaptations may become apparent to those skilled in the relevant arts in view of the foregoing description, when read in conjunction with the accompanying drawings and the appended claims. However, all such and similar modifications of the teachings will still fall within the scope as defined in the appended claims.

## Claims

1. A method comprising:
determining a pulse time based on a timestamp associated with a feature within a first waveform sensed by a first wearable device on a first region of the user and a timestamp associated with the feature within a second waveform sensed by a second wearable device on a second region of the user; and
authenticating the user based on the pulse time.

2. The method of claim 1, wherein the first waveform is a first photoplethysmography waveform and the second waveform is a second photoplethysmography waveform.

3. The method of claim 1 or claim 2, wherein the feature comprises at least one of:
a start of beat;
a systolic peak;
a dicrotic notch;
a diastolic peak; or
an end of beat.

4. The method of any of claims 1 to 3, wherein the method comprises:
receiving the first waveform ;
determining the timestamp associated with the feature within the first waveform;
receiving the second waveform; and
determining the timestamp associated with the feature within the second photoplethysmography waveform;

5. The method of any of claims 1 to 4, wherein the method comprises:
receiving the timestamp associated with the feature within the first waveform; and
receiving the timestamp associated with the feature within the second waveform.

6. The method of any of claims 1 to 5, wherein authenticating the user based on the pulse time comprises:
determining that the pulse time matches a reference pulse time; and
authenticating the user based on the pulse time matching the reference pulse time.

7. The method of claim 6, wherein the method comprises:
determining the reference pulse time based on a timestamp of the feature within a previous first waveform sensed by the first wearable device on the first region of the user and a timestamp of the feature within a previous second waveform sensed by the second wearable device on the second region of the user.

8. The method of claims 6 or claim 7, wherein the method comprises:
updating the reference pulse time based on the pulse time.

9. The method of any of claims 1 to 7, wherein the first wearable device and the second wearable device are time synchronized.

10. The method of any of claims 1 to 8, wherein the method comprises:
determining a first inter-beat interval based on a timestamp associated with the feature within the first waveform wearable device and a timestamp associated with a consecutive feature within the first waveform wearable device;
determining a second inter-beat interval based on a timestamp associated with a feature within the second waveform sensed by the second wearable device on the second region of the user and a timestamp associated with a consecutive feature within the second waveform wearable device; and
authenticating the user based on the first inter-beat interval and the second inter-beat interval.

11. The method of claim 10, wherein authenticating the user based on the first inter-beat interval and the second inter-beat interval comprises:
determining that the first inter-beat interval matches the second inter-beat interval; and
authenticating the user based the first inter-beat interval matching the second inter-beat interval.

12. The method of any of claims 1 to 11, wherein the method comprises:
unlocking the second wearable device.

13. The method of any of claims 1 to 12, wherein at least one of the first wearable device and the second wearable device comprise:
a phone;
a watch;
a ring;
a wrist band;
an arm band;
a chest strap;
an earbud;
a garment;
a headset;
a head mounted visor; or
glasses.

14. An apparatus comprising means for performing the method of any of claims 1 to 13.

15. A computer program comprising instructions, which, when executed by an apparatus, cause the apparatus to perform the method of any of claims 1 to 13.
